# EUROPEAN PATENT APPLICATION

(11) **EP 4 485 154 A1**
(43) Date of publication of application: **01.01.2025**
(21) Application number: 24184352.3
(22) Date of filing: 25.06.2024
(51) Int. Cl.: G06F 3/0362, G06F 3/0354, A61B 5/12, A61B 5/00, G06F 1/16, G06F 3/04847, G06F 3/04886

(54) **CONTROL DEVICE FOR AUDIOMETER**

(30) Priority: 26.06.2023 EP 23181442
(71) Applicant: Interacoustics A/S, 5500 Middelfart (DK)
(72) Inventor: NIELSEN, Lillian, DK-5500 Middelfart (DK); FRANK, Søren, DK-5500 Middelfart (DK); APITZ, Nikolaj, DK-5500 Middelfart (DK); CRAMER, Thomas, DK-5500 Middelfart (DK)
(74) Representative: Demant

(57) **Abstract**

The present application relates to a control device, the control device comprising a housing, and at least one dial comprising a rotatable part, where said housing comprises a touch screen comprising a first user input unit and a user output unit both forming part of a user interface presented on said touch screen, the first user input unit configured to receive a touch input from a user and providing a corresponding first input signal and the user output unit configured to provide an output on said touch screen based on an output signal, where said at least one dial comprising a second user input unit, said dial is configured to control magnitude and/or size and/or intensity of one or more audiological parameters of an audiological test via said rotatable part, and where said second user input unit is configured to provide a second input signal in response to the user rotating said rotatable part, wherein said control device further comprising a communication module for establishing a communication link between the control device and at least one auxiliary device, said communication link providing that information and/or data is transmitted to and/or received from said auxiliary device, and wherein said user interface configured to provide one of a plurality of user interface layouts presentable on said touch screen, and where said user interface is configured to adaptively adjust/modify said provided user interface layout in response to said first and/or second input signals.

## Description

### SUMMARY

The present application relates to a control device.

The present application further relates to a system comprising a control device and an audiological test instrument.

The present application further relates to a method of operating one or more types of audiological tests.

The present application further relates to a computer program comprising instructions which, when the program is executed by a control device or a system, cause the control device or system to carry out the method.

### A control device

Traditional standalone audiometers usually have several physical pressure keys as well as two dials each including a rotatable wheel to enable a user, such as a hearing care professional, to carry out a hearing test on a patient (i.e., a test subject). To make it easy for the user to find the functions they need for a specific hearing test, the number of buttons and therefore also the size of the audiometer may be high/large. However, this can lead to a complex user flow. Also, to enable use of the audiometer in a number of different countries and regions around the world, a variety of different designs and labelling of the keys and dials may be needed.

As a consequence of the development going from standalone based audiometers to PC based audiometers, the users are requesting an audiometer keyboard instead of the PC based keyboard. This is as the PC based keyboards are harder to use due to is many keys dedicated for other functions than required for controlling the audiometer. Further, the two dials on an audiometer keyboard are the preferred way to operate the two channels of the audiometer, which further decreases the usefulness of the PC based keyboard.

Therefore, there is a need for an audiometer keyboard that enables an easy user flow.

In an aspect of the present application, a control device is provided.

In an aspect of the present application, a keyboard is provided.

For example, the control device may be a keyboard, such as an audiometer keyboard.

The control device (or keyboard) may be suitable for operating one or more types of audiological tests on a test subject.

The control device (or keyboard) may be suitable for interacting/communicating with an auxiliary device, such as an audiological test instrument.

The control device (or keyboard) may be suitable for remotely controlling an auxiliary device.

The control device (or keyboard) may comprise a housing.

The housing may contain/enclose at least part of the elements of the control device (or keyboard). The housing may form at least part of the outer shell of the control device (or keyboard).

The control device (or keyboard) may comprise at least one dial comprising a rotatable part. The control device (or keyboard) may comprise two dials each comprising a rotatable part. For example, said rotatable part may be a rotatable wheel.

The housing may comprise a touch screen.

The touch screen may comprise a touch panel e.g., a capacitive touch screen panel, and a display element.

The touch screen may comprise a first user input unit. The first user input unit may comprise the touch panel.

The touch screen may comprise a user output unit. The user output unit may comprise the display element.

For example, the touch panel may be layered on the top of the (electronic visual) display element of the control device (or keyboard).

The first user input unit and the user output unit may form part of a user interface.

The user interface may be presented/provided on said touch screen (at least during operation of said control device (or keyboard)).

The first user input unit may be configured to receive a touch input from the user.

The touch input may be provided via the user interface.

The first user input unit may be configured to provide a corresponding first input signal. In other words, the first user input unit may be configured to provide a first input signal in response to receiving a touch input from the user.

For example, when a user touches the touch screen the first user input unit may detect the touch as a touch input and generate a corresponding first input signal.

The first input signal may at least comprise information about position, movement, and/or duration of the touch input from the user (on the touch panel). In other words, the first input signal may comprise data including at least positional, movement and/or durational input parameters.

The user output unit may be configured to provide an output on said touch screen based on an output signal. In other words, the user output unit may provide an output on the display element in response to receiving an output signal. In other words, the user interface may display an output on the touch screen in response to receiving an output signal.

The at least one dial may comprise a second user input unit.

The dial may be configured to control magnitude and/or size and/or intensity of one or more audiological parameters of an audiological test via said rotatable part.

The second user input unit may be configured to provide a second input signal in response to the user rotating said rotatable part.

For example, the user may rotate the rotatable part to e.g. increase or decrease intensity of a stimulus signal provided to a patient during an audiological hearing test. In response to the rotation, the second user input unit may generate a second input signal.

The control device (or keyboard) may further comprise a communication module.

The communication module may comprise an antenna and a transceiver circuitry for establishing a communication link.

The communication module may be suitable for establishing a communication link between the control device (or keyboard) and at least one auxiliary device.

The communication link may provide/facilitate that information and/or data is transmitted to and/or received from said auxiliary device.

The communication link may be a wireless communication link.

The wireless link may be based on Bluetooth technology (e.g., Bluetooth Low-Energy technology), or Ultra WideBand (UWB) technology.

For example, the information and/or data may comprise configuration data enabling an audiological test instrument to modulate and/or adjust an acoustic stimulus and present it to a patient.

The user interface may be configured to provide one of a plurality of user interface layouts.

User interface layouts may refer to user interface designs and/or user interface configurations.

The user interface layouts may be presentable on said touch screen (display element).

The user interface may be configured to adaptively adjust/modify said provided user interface layout.

The user interface may be configured to adaptively adjust/modify said provided user interface layout in response to the first input signal and/or in response to the second input signal.

The control device (or keyboard) may comprise a processor.

The processor may be connected to (communicating with) the first user input unit and the second user input unit.

The processor may be configured to receive a first input signal from said first user input unit.

The processor may be configured to receive a second input signal from said second user input unit.

The processor may be connected to (communicating with) the user output unit.

The processor may be configured to send an output signal to said user output unit.

The control device (or keyboard) may comprise a memory.

The memory may be connected to (communicating with) the processor.

The memory may be configured to store a plurality of user interface layouts.

Therefore, a control device (or keyboard) which provides an easy user flow for controlling an auxiliary device, such as an audiological test instrument, is provided.

The auxiliary device may be an audiological test instrument.

The auxiliary device may be a computing device connected to an audiological test instrument. Accordingly, the control device (or keyboard) may function as a control device (or keyboard) for (remotely controlling) an audiological test instrument and/or for (remotely controlling) a computing device connected to an audiological test instrument.

Each of said user interface layouts may display an option for selecting one out of a plurality of types of audiological tests and corresponding user interface layout.

The option may be represented by a button or menu on the user interface layouts. For example, the user may touch the button or menu presented on the user interface in order to select a type of audiological test.

For example, each user interface layout may comprise e.g., a drop-down menu which lists a plurality of types of audiological tests. The user may have the possibility of selecting one of the types of audiological tests.

For example, a plurality of types of audiological tests may refer to a pediatric test, or may refer to a hearing test, such as a pure tone assessment test or a speech audiometry test.

Each of the user interface layouts may be configured to present/display options each representing a parameter selectable by a user.

The options may be related to the type of audiological test selected by the user.

For example, each type of audiological test may comprise parameters that are common to all the test types, e.g., intensity, channel selection, etc, but may also comprise parameters that are specific to that type of test selected.

The parameters may comprise ear selection.

The parameters may comprise transducer selection.

The parameters may comprise stimulus type.

The parameters may comprise frequency level.

The parameters may comprise channel selection.

The control device (or keyboard) may be configured to transmit the information and/or data via the communication link to the auxiliary device in response to the user providing an input to the first user input unit.

The control device (or keyboard) may be configured to transmit the information and/or data via the communication link to the auxiliary device in response to the user providing an input to the second user input unit.

The at least one dial may be arranged at an outer edge of said housing.

The at least one dial may protrude at least partly from said outer edge.

The rotatable wheel of the at least one dial may be arranged at an angle relative to a plane of the touch screen.

For example, the dial may comprise a first longitudinal axis extending from a first end to a second end of said dial. When the control device (or keyboard) is placed on a table, the first end of the dial may rest on the table thereby increasing the stability of the control device (or keyboard) on the table. The second end of the dial may be located at distance from the table, and said distance may be larger than a distance an adjacently placed part of the housing (and therefore of the touch screen) is located from the table. Thereby, the dial may protrude from said housing both along said first longitudinal axis and perpendicular to said first longitudinal axis.

For example, the rotatable wheel may be arranged at the second end of said dial. The rotatable wheel may have an axis of rotation parallel to said first longitudinal axis. Thereby, the rotatable wheel may be configured to rotate in a plane perpendicular to said first longitudinal axis.

The dial may comprise at least one key (e.g., a push-button), where said at least one key is dedicated for operating one predefined function of said type of audiological test.

For example, the dial may comprise a first key, such as a primary key, and a second key, such as a secondary key. Each of the first key and the second key may be dedicated for controlling an essential function of the type of audiological test chosen by the user.

For example, a function to be controlled by the first or second key may be a store function or a talk forward function.

The at least one key may be configured to operate a store function. For example, the store function may be for storing a present parameter setting.

The at least one key may be configured to operate an activation of a talk forward function. The at least one key may be configured to operate a deactivation of a talk forward function.

A talk forward function may be configured to enable the user to communicate with said patient. The user interface may be deactivated for user input in response to activation of said talk forward function. The user interface may be activated for user input in response to deactivation of said talk forward function.

The at least one rotatable wheel may be configured to provide feedback to the user in response to the user rotating the rotatable wheel.

Feedback may refer to providing audio feedback (e.g., via a loudspeaker), visual feedback (e.g., via a light), or haptic feedback.

Feedback may refer to amending the functionality of the rotatable wheel. For example, amending the functionality of the rotatable wheel may include adjusting the resistance required for the user to rotate the wheel.

For example, the rotatable wheel may be configured to adjust the intensity of channel 1. At the level where an extended intensity range is reached, a light indicator may provide visual feedback around the wheel (e.g., by flashing a number of times). Additionally, or alternatively, when an extended intensity range is reached, turning the rotatable wheel further in the same direction may require that you use more force to turn the wheel.

The control device (or keyboard) may comprise a light (light indicator) arranged adjacent each of the at least one rotatable wheel. The light (light indicator) may be configured to change colour, intensity, and/or appearance of the light depending on a control signal from the processor.

The control device (or keyboard) may comprise two dials, where each dial may comprise a rotatable wheel.

Each dial may be configured for operating a channel of said audiological test instrument. For example, one channel may be configured for presenting a stimulus signal to the ear(s) of the test subject.

For example, one channel may be configured for presenting a masking signal to the ear(s) of the test subject.

The control device (or keyboard) may be configured to activate one or both channels at the same time. Thereby, it may be the case that only a channel for presenting a stimulus signal is activated. It may also be the case that one channel presents a stimulus signal, and another channel presents a masking signal for masking the stimulus signal.

### A system

In a further aspect, a system comprising a control device (or keyboard) and an audiological test instrument as described above, in the `detailed description' and in the claims, is moreover provided.

The system may be configured to establish a communication link between the control device (or keyboard) and the audiological test instrument. The communication link may provide that information and/or data is transmitted between said control device (or keyboard) and said audiological test instrument.

### A method

In an aspect, a method of operating one or more types of audiological tests is furthermore provided by the present application.

The method may comprise providing a control device (or keyboard) comprising a housing and at least one dial.

The method may comprise providing a first user input unit and a user output unit both forming part of a user interface presented on a touch screen of said housing.

The method may comprise receiving a touch input from a user via the first user input unit. The method may comprise providing a corresponding first input signal.

The method may comprise providing an output on said touch screen based on an output signal. The output on said touch screen may be provided via the user output unit.

The method may comprise providing a second user input unit on said at least one dial.

In other words, the at least one dial may comprise a second user input unit for detecting that the user touches (e.g., rotates) the dial.

The method may comprise controlling magnitude and/or size and/or intensity of one or more audiological parameters of said audiological test via a rotatable part of said at least one dial.

The method may comprise providing a second input signal in response to the user rotating said rotatable part.

The method may comprise transmitting information and/or data to an auxiliary device via a communication link.

The method may comprise receiving information and/or data from an auxiliary device via a communication link.

The method may comprise providing one of a plurality of user interface layouts on said touch screen.

The method may comprise adaptively adjusting/modifying said provided user interface layout in response to said first and/or second input signals.

It is intended that some of or all the structural features of the control device (or keyboard) and the system described above, in the `detailed description of embodiments' or in the claims, can be combined with embodiments of the method, when appropriately substituted by a corresponding process and vice versa. Embodiments of the method have the same advantages as the corresponding control device (or keyboard) and/or system.

### Use

In an aspect, use of a control device (or keyboard) and a system as described above, in the `detailed description' and in the claims, is moreover provided.

### A computer program

A computer program (product) comprising instructions which, when the program is executed by a computer, e.g., a control device (or keyboard) or a system (as disclosed above), cause the computer to carry out (steps of) the method described above and in the claims is furthermore provided by the present application.

A computer program (product) comprising instructions which, when the program is executed by a control device (or keyboard), comprising a touch screen, a processor for receiving a first input signal from a first user input unit and a second input signal from a second user input unit, and a communication module, cause the control device (or keyboard) to function as a control device (or keyboard) (as disclosed above) for an auxiliary device, is furthermore provided by the present application.

The instructions may cause the control device (or keyboard) to translate at least said first and second input signals into information and/or data.

The instructions may cause the control device (or keyboard) to send information and/or data to an auxiliary device.

The information and/or data may comprise at least said first and second input signals.

The first and second input signals can be interpreted by the auxiliary device as one of a plurality of potential commands in a provided one of a plurality of user interface layouts.

The auxiliary device may be configured for interpreting said first and second input signals in combination with said provided user interface layout to determine an appropriate command for the provided user interface layout, and updating (e.g., adaptively adjust/modify) the user interface layout in response to said appropriate command.

The instructions may cause the control device (or keyboard) to receive the appropriate command from said auxiliary device.

The instructions may cause the control device (or keyboard) to adaptively adjust/modify the provided user interface layout in response to the appropriate command.

The adaptive adjustment/modification may be carried out via the processor and/or a control unit.

The adaptive adjustment/modification may be carried out at least partly based on parameters stored in the memory.

For example, the parameters stored in the memory may represent ear selection, transducer selection, stimulus type, frequency level, and/or channel selection.

### A data-processing system

In an aspect, a data-processing system comprising a processor and program-code means for causing the processor to perform at least some (such as a majority or all) of the steps of the method described above, and in the claims, is furthermore provided by the present application.

### An APP

In a further aspect, a non-transitory application, termed an APP, is furthermore provided by the present application. The APP comprises executable instructions configured to be executed on a control device (or keyboard) to implement a user interface of the control device (or keyboard) described above in the `detailed description', and in the claims.

### BRIEF DESCRIPTION OF DRAWINGS

The aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features, and/or technical effect will be apparent from and elucidated with reference to the illustrations described hereinafter in which:
FIG. 1 shows an exemplary system according to the present application.
FIG. 2 shows an exemplary control device (or keyboard) according to the present application.
FIG. 3 shows an exemplary control device (or keyboard) according to the present application, seen from the side.
FIG. 4 shows an exemplary block diagram of the control device (or keyboard) according to the present application.
FIG. 5 shows an exemplary control device (or keyboard) according to the present application, seen in perspective.
FIG. 6 shows an exemplary control device (or keyboard) according to the present application, seen from the back.

The figures are schematic and simplified for clarity, and they just show details which are essential to the understanding of the disclosure, while other details are left out. Throughout, the same reference signs are used for identical or corresponding parts.

Further scope of applicability of the present disclosure will become apparent from the detailed description given hereinafter. However, it should be understood that the detailed description and specific examples, while indicating preferred embodiments of the disclosure, are given by way of illustration only. Other embodiments may become apparent to those skilled in the art from the following detailed description.

### DETAILED DESCRIPTION

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the control device (or keyboard), system, and method are described by various blocks, functional units, modules, components, circuits, steps, processes, algorithms, etc. (collectively referred to as "elements"). Depending upon a particular application, design constraints or other reasons, these elements may be implemented using electronic hardware, computer program, or any combination thereof.

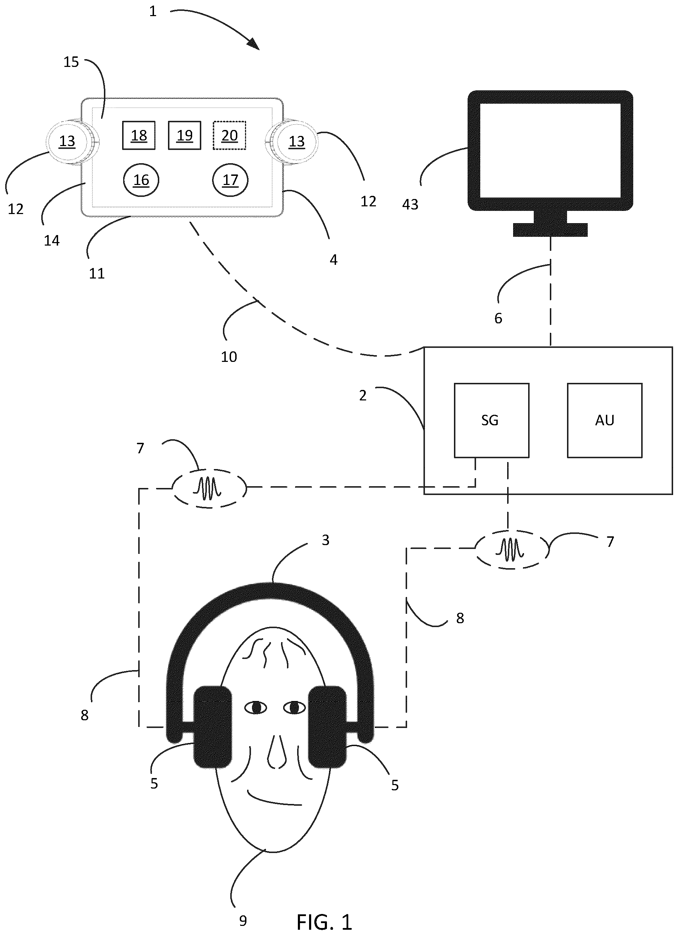

FIG. 1 shows an exemplary system according to the present application.

The system 1 is shown to comprise an audiological test instrument 2, an audio device 3, a monitor 43, and a control device (or keyboard) 4.

The audiological test instrument 2 may comprise a stimulus generator SG and an analysis unit AU.

The audio device 3 may comprise at least one audio output unit 5. In the example of FIG. 1, the audio device 3 is shown to have two audio output units 5.

The monitor 43 may be connected to the audiological test instrument 2 via either a wired or wireless connection 6 so that the monitor can present the software program for running an audiological test.

The stimulus generator SG may be configured to generate at least one stimulus 7, such as a stimulus signal and a masking signal, and to transmit said at least one stimulus to the audio device 3.

For example, the stimulus generator SG may be configured to provide a plurality of consecutive stimuli 7 according to a test protocol of a specific (chosen) test type.

The stimulus generator SG may transmit the stimuli 7 via a wired or wireless connection 8 to the audio device 3.

In FIG. 1, the audio device 3 is illustrated as being a headset that is placed at the ears of a test subject 9, but the audio device 3 may instead be placed at/in only one of the ears, and other earphones or other devices comprising a transducer may alternatively be used. The two output units 5 of the audio device 3 may (each) comprise a transducer, and the acoustic output units 5 may each be configured to provide the at least one stimulus into the ear(s) of the test subject 9 via said transducer. Accordingly, consecutive stimuli 7 may be transmitted to the audio device 3 and each of the output units 5 may present the stimuli 7 into the ears of the test subject 9 via said transducers.

The analysis unit AU may be suitable for analysing e.g., a correspondence between each of the provided stimuli 7 and a following test subject input, e.g., detected by the auxiliary device such as a test subject remote control or as expressed verbally by the test subject.

The analysis unit AU may be configured to adjust the test protocol of the selected test type according to said analysed correspondence.

The control device (or keyboard) 4 may be connected to the audiological test instrument 2 via either a wired or wireless connection 10. In case of a wireless connection 10, the control device (or keyboard) 4 can be moved around by the user operating the audiological test instrument 2.

The control device (or keyboard) 4 may comprise a housing 11 and at least one dial 12 comprising a rotatable part 13. In FIG. 1, the control device (or keyboard) 4 comprises two dials 12.

The housing 11 of the control device (or keyboard) 4 is shown to comprise a touch screen 14 presenting a user interface 15. The user interface 15 is shown to present a plurality of parameters or objects on the touch screen 14.

The parameters or objects may either represent a parameter selectable by the user and giving the user the possibility of amending the size/magnitude of, or may present the current settings and type of test selected by the user. For example, the parameters or objects may represent ear selection, transducer selection, stimulus type, frequency level, and/or channel selection.

For example, the parameters (e.g., options/buttons/menus presented on the user interface) may represent an option for the user to select that a stimulus is transmitted in a first 16 and/or a second channel 17. The parameters may additionally, or alternatively, represent a button 18 between a plurality of user interface layouts, and/or represent a button 19 the type of audiological test to be carried out. Further options/buttons/menus 20 representing other selections relating to the test may be presented.

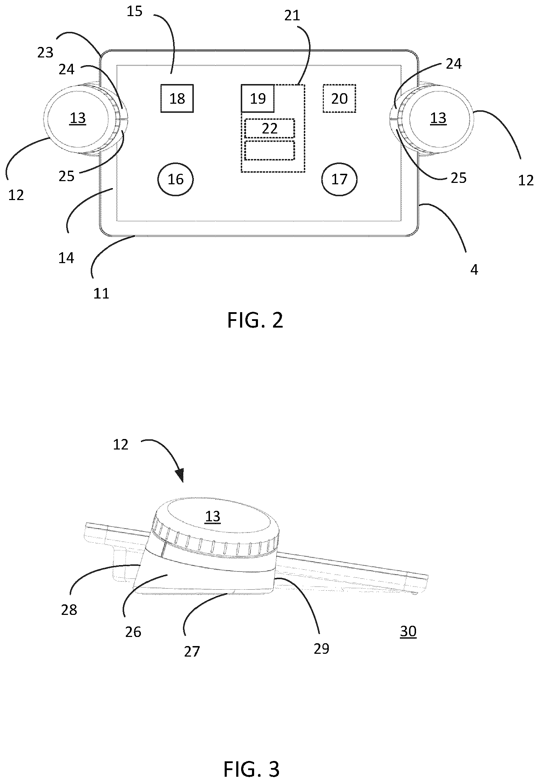

FIG. 2 shows an exemplary control device (or keyboard) according to the present application.

In FIG. 2, the control device (or keyboard) as also shown in FIG. 1, is seen from above in more detail. For similar features as shown in FIG. 1, similar reference numbers are used.

It is shown that one or more of the parameters, exemplified by the button 19 on the user interface 15 for selecting the type of audiological test to be carried out, may comprise a drop-down menu 21 listing a number of further options/buttons/menus 22 selectable by the user. For example, the options/buttons/menus 22 may provide the user the option of selecting a pediatric test or a hearing test, such as a pure tone assessment test or a speech audiometry test.

The touch screen 14 may comprise a first user input unit and a user output unit both forming part of a user interface 15 presented on the touch screen 14. The first user input unit may be configured to receive a touch input from the user, when the user touches the (e.g., capacitive) touch panel of the touch screen 14. The first input unit may generate a corresponding first input signal that may be send to a processor of the control device (or keyboard).

The touch screen 14 may comprise a user output unit configured to provide an output on a display element of the touch screen 14 based on an output signal.

Each of the two dial 12 is shown to be arranged at an outer edge 23 of said housing 4 and protruding at least partly from said outer edge 23.

In the example of FIG. 1, each of the two dials 12 comprises a first key 24 and a second key 25 each dedicated for operating one predefined function of the selected type of audiological test. The first key 24 could for example be configured to operate a store function for storing a present parameter, and the second key 25 could for example be configured to operate activation/deactivation of a talk forward function enabling the user to communicate with a test subject. The user interface 15 can be deactivated for further user input in response to activation of said talk forward function.

The user interface 15 may present/display one of a plurality of user interface layouts on the touch screen 14. Each of the user interface layouts presents/displays parameters selectable by the user, where the parameters are related to the type of audiological test selected by said user. In other words, when the user selects a specific type of audiological test, a user interface layout specifically designed for the selected test is presented, and a number of parameters and objects related to the selected test is displayed, e.g., so that the user can activate/deactivate a parameter and/or adjust the size of the parameter.

FIG. 3 shows an exemplary control device (or keyboard) according to the present application, seen from the side. The control device (or keyboard) is in a resting position on e.g., a table.

For similar features as shown in FIGS. 1 and 2, similar reference numbers are used.

In FIG. 3, a dial 12 is seen from the side. The dial 12 is shown to comprise the rotatable part 13 arranged on top of a tower part 26.

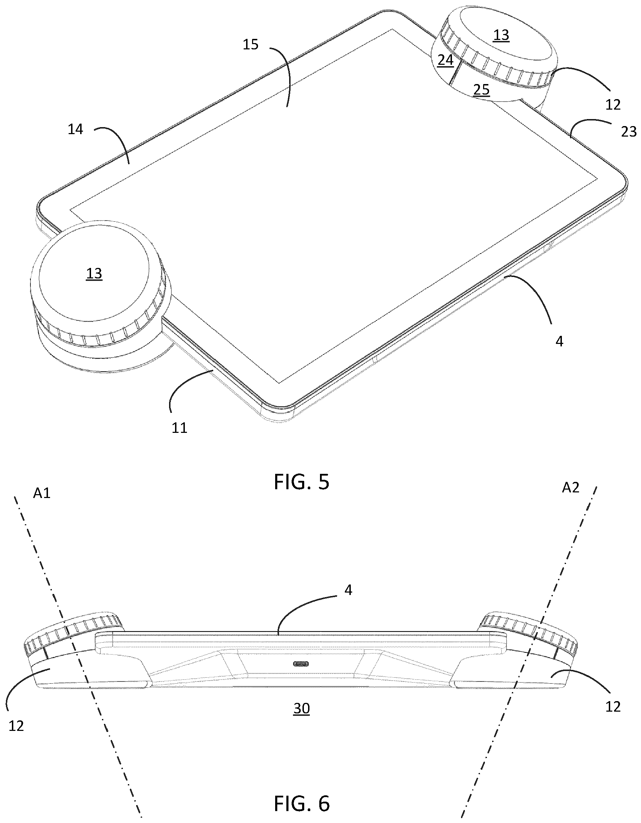

The tower part 26 of the dial 12 may have a plane lower surface 27 contacting a surface e.g., a table, when the control device (or keyboard) 4 is resting on the surface thereby supporting the stability of the control device (or keyboard) 4. It is shown that the sides of the tower part 26 may have a conical frustum shape, other shapes, such as tubular, oval, or many edged shapes, are foreseen. Accordingly, it is shown that the tower part 26 may have a front surface part 28 leaning with a smaller angle than a back surface part 29 relative to the plane lower surface 27. As also shown in FIG. 3, the dial (i.e., both the tower part 26 and the rotatable part 13) is leaning away from the touch screen 14. In other words, a longitudinal axis (see FIG. 6) of the dial 12 has an angle relative to a norm of a surface 30 on which the control device (or keyboard) 4 is placed.

Thereby, the dial 12 is angled towards the user and away from the touch screen 14, which facilitates an ergonomic position of the user's hands.

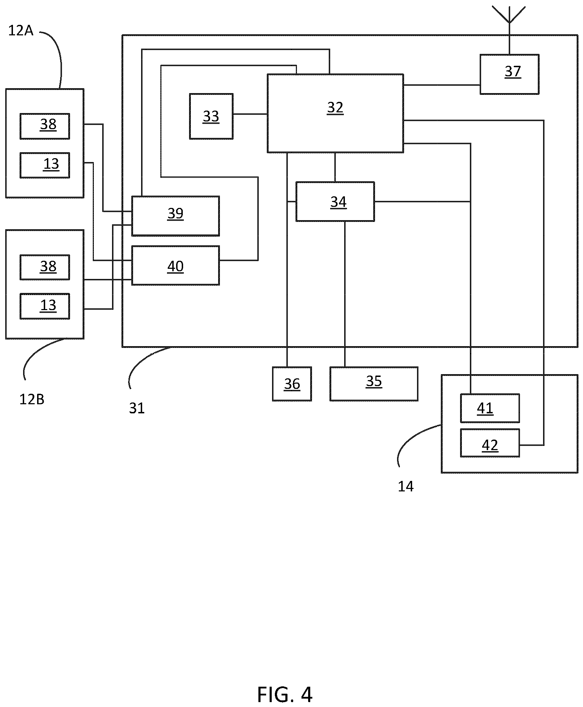

FIG. 4 shows an exemplary block diagram of the control device (or keyboard) according to the present application. The lines indicate the connections/communication lines between the elements in the block diagram.

The control device (or keyboard) may comprise a mainboard 31 comprising a processor 32 and a memory 33 for storing parameters.

The control device (or keyboard) may further comprise a power controller 34 connected to a battery 35 and a power supply 36 for providing power e.g., via a USB cable.

The control device (or keyboard) may further comprise a communication module 37 for establishing a communication link (wireless or wired) between the control device (or keyboard) and at least one auxiliary device, where the communication link provides that information and/or data is transmitted to and/or received from said auxiliary device.

A first dial 12A and a second dial 12B may each comprise at least one key 38 and a rotatable part 13. The at least one keys 38 and rotatable parts 13 together with a encoder filter 39 and button logic and debounce 40 form part of a second user input unit for providing a second input signal to the processor 32.

A touch screen 14 may comprise a touch panel 41 forming part of a first user input unit for providing a first input signal in response to receiving a touch input from the user.

The touch screen 14 may further comprise a display element 42 forming part of a user output unit configured to provide an output on the display element 42 of the touch screen 14 in response to receiving an output signal from the processor 32.

FIG. 5 shows an exemplary control device (or keyboard) according to the present application, seen from in perspective. The control device (or keyboard) is in a resting position on e.g., a table.

For similar features as shown in the previous FIGS., similar reference numbers are used.

FIG. 6 shows an exemplary control device (or keyboard) according to the present application, seen from the back. The control device (or keyboard) is in a resting position on e.g., a table.

For similar features as shown in the previous FIGS., similar reference numbers are used.

In FIG. 6, a longitudinal axis A1, A2 of the two dials 12 are shown. In each case it is shown that the longitudinal axes A1, A2 are arranged with an angle relative to a norm of a surface 30 on which the control device (or keyboard) 4 is placed. Thus, the dials 12 are angled away from the touch screen 14.

It is intended that the structural features of the instrument described above, either in the detailed description and/or in the claims, may be combined with steps of the method, when appropriately substituted by a corresponding process.

As used, the singular forms "a," "an," and "the" are intended to include the plural forms as well (i.e., to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that, when an element is referred to as being "connected" or "coupled" to another element, it can be directly connected or coupled to the other element, but an intervening element may also be present, unless expressly stated otherwise. Furthermore, "connected" or "coupled", as used herein, may include wirelessly connected or coupled. As used herein, the term "and/or" includes any and all combinations of one or more of the associated listed items. The steps of any disclosed method are not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

The claims are not intended to be limited to the aspects shown herein but are to be accorded the full scope consistent with the language of the claims, wherein reference to an element in the singular is not intended to mean "one and only one" unless specifically so stated, but rather "one or more." Unless specifically stated otherwise, the term "some" refers to one or more.

## Claims

1. Control device, the control device comprising:
- a housing, and
- at least one dial comprising a rotatable part,
- where said housing comprises a touch screen comprising a first user input unit and a user output unit both forming part of a user interface presented on said touch screen, the first user input unit configured to receive a touch input from a user and providing a corresponding first input signal and the user output unit configured to provide an output on said touch screen based on an output signal,
- where said at least one dial comprising a second user input unit, said dial is configured to control magnitude and/or size and/or intensity of one or more audiological parameters of an audiological test via said rotatable part, and where said second user input unit is configured to provide a second input signal in response to the user rotating said rotatable part,
- wherein said control device further comprising a communication module for establishing a communication link between the control device and at least one auxiliary device, said communication link providing that information and/or data is transmitted to and/or received from said auxiliary device, and
- wherein said user interface configured to provide one of a plurality of user interface layouts presentable on said touch screen, and where said user interface is configured to adaptively adjust/modify said provided user interface layout in response to said first and/or second input signals.

2. Control device according to claim 1, wherein said auxiliary device is an audiological test instrument or a computing device connected to an audiological test instrument.

3. Control device according to any one of the preceding claims, wherein each of said user interface layouts displays an option for selecting one out of a plurality of types of audiological tests and corresponding user interface layout.

4. Control device according to any one of the preceding claims, wherein each of said user interface layouts are configured to present options each representing a parameter selectable by a user, where the options are related to the type of audiological test selected by said user.

5. Control device according to claim 4, wherein said parameters comprises ear selection, transducer selection, stimulus type, frequency level, and/or channel selection.

6. Control device according to any one of the preceding claims, wherein said control device is configured to transmit said information and/or data via the communication link to said auxiliary device in response to the user providing an input to the first user input unit.

7. Control device according to any one of the preceding claims, wherein said at least one dial is arranged at an outer edge of said housing and protruding at least partly from said outer edge.

8. Control device according to any one of the preceding claims, wherein said rotatable wheel of said at least one dial is arranged at an angle relative to a plane of the touch screen.

9. Control device according to any one of the preceding claims, wherein said dial comprises at least one key, where said at least one key is dedicated for operating one predefined function of said type of audiological test.

10. Control device according to claim 9, wherein one key is configured to operate a store function for storing a present parameter setting, and/or one key is configured to operate activation/deactivation of a talk forward function enabling the user to communicate with said test subject, wherein said user interface layout is deactivated for user input in response to activation of said talk forward function.

11. Control device according to any one of the preceding claims, wherein the at least one rotatable wheel is configured to provide feedback to the user in response to the user rotating said wheel.

12. Control device according to any one of the preceding claims, wherein said control device comprises two dials, where each dial is configured for operating a channel of said audiological test instrument.

13. Control device according to any one of the preceding claims, wherein the communication link is a wireless communication link.

14. System comprising
- a control device according to any one of the claims 1-13, and
- an audiological test instrument,
- wherein the system is configured to establish a communication link between the control device and the audiological test instrument, said communication link providing that information and/or data is transmitted between said control device and said audiological test instrument.

15. Method of operating one or more types of audiological tests, the method comprising:
- providing a control device comprising a housing and at least one dial,
- providing a first user input unit and a user output unit both forming part of a user interface presented on a touch screen of said housing,
- receiving a touch input from a user via the first user input unit, and providing a corresponding first input signal,
- providing an output on said touch screen based on an output signal, via the user output unit,
- providing a second user input unit on said at least one dial,
- controlling magnitude and/or size and/or intensity of one or more audiological parameters of said audiological test via a rotatable part of said at least one dial,
- providing a second input signal in response to the user rotating said rotatable part,
- transmitting information and/or data to, and/or receiving information and/or data from an auxiliary device via a communication link, and
- providing one of a plurality of user interface layouts on said touch screen, and
- adaptively adjusting/modifying said provided user interface layout in response to said first and/or second input signals.

16. A computer program comprising instructions which, when the program is executed by a control device of any of claims 1-13 or a system of claim 14, cause the control device or system to carry out the method according to claim 15.

17. A computer program product comprising instructions which, when the program is executed by a control device comprising a touch screen, a processor for receiving a first input signal from a first user input unit and a second input signal from a second user input unit, and a communication module, cause the control device to function as a control device for an auxiliary device, in that the instructions cause the control device to
- translate at least said first and second input signals into information and/or data,
- send information and/or data to an auxiliary device, said information and/or data comprising at least said first and second input signals, where said first and second input signals can be interpreted by the auxiliary device as one of a plurality of potential commands in a provided one of a plurality of user interface layouts, where the auxiliary device is configured for interpreting said first and second input signals in combination with said provided user interface layout to determine an appropriate command for the provided user interface layout, and updating the user interface layout in response to said appropriate command,
- receive said appropriate command from said auxiliary device, and
- adaptively adjust/modify said provided user interface layout in response to said appropriate command.
